# EUROPEAN PATENT APPLICATION

(11) **EP 4 151 745 A1**
(43) Date of publication of application: **22.03.2023**
(21) Application number: 21882957.0
(22) Date of filing: 21.04.2021
(51) Int. Cl.: C12Q 1/48, C12Q 1/54, C12N 9/10

(54) **NOVEL ENZYME ACTIVITY MEASUREMENT METHOD**

(30) Priority: 19.10.2020 KR 20200135340
(71) Applicant: CJ Cheiljedang Corporation, Seoul 04560 (KR)
(72) Inventor: KIM, Jungeun, Seoul 04560 (KR); CHU, Sun, Seoul 04560 (KR); PARK, Sunghee, Seoul 04560 (KR); BYUN, Sung Bae, Seoul 04560 (KR); JU, Jae Yeong, Seoul 04560 (KR)
(74) Representative: Heubeck, Christian
(86) International application number: PCT/KR2021/005035
(87) International publication number: WO 2022/085882

(57) **Abstract**

Provided are a method of selecting dextransucrase having transglycosylation activity for glucose acceptors among enzymes, particularly dextransucrases, and a method of measuring activity thereof.

## Description

### [Technical Field]

The present disclosure relates to a method of selecting dextransucrase having transglycosylation activity for glucose acceptors among enzymes, particularly dextransucrases, and a method of measuring activity thereof.

### [Background Art]

Dextransucrase releases glucose from sugar, and at the same time, catalyzes a polymer polymerization reaction of glucose, thereby producing polysaccharides such as dextrans or oligosaccharides, which are polymeric materials. Otherwise, when substances such as stevia (steviol glycoside), polyphenol, *etc.* are present in a substrate, dextransucrase releases glucose from sugar due to its broad substrate specificity, and then exhibits transglycosylation activity capable of transferring glucose to the stevia, polyphenol, *etc.* In this regard, the transglycosylated stevia, polyphenol, *etc.* may have improved industrial value such as improved solubility, improved taste, *etc.*

The above-described activity of dextransucrase is generally measured by determination of reducing sugar (DNS), which analyzes how much sugar is hydrolyzed into fructose and glucose by measuring free fructose. However, the above method may measure only the degree of hydrolysis of sugar by dextransucrase, and has a problem in that it does not reflect transglycosylation activity. In other words, when improved dextransucrase is selected using DNS, it is highly likely to select only dextransucrase having excellent sugar hydrolysis or dextran polymerization activity, not dextransucrase having excellent transglycosylation activity.

However, despite the above problems, there has been no method other than DNS in measuring the dextransucrase activity, and there has been a need to develop a novel method of measuring the dextransucrase activity.

### [Disclosure]

### [Technical Problem]

The present inventors have developed a high-speed selection method of quickly and efficiently selecting or improving dextransucrase having transglycosylation activity for glucose acceptors by measuring a difference between the amount of free glucose after a sugar hydrolysis reaction by dextransucrase and the amount of free glucose after a sugar hydrolysis reaction and a transglycosylation reaction, thereby completing the present disclosure.

### [Technical Solution]

The present disclosure provides a method of selecting dextransucrase having transglycosylation activity for glucose acceptors, the method including the steps of (i) determining the amount of free glucose by reacting sugar with dextransucrase; (ii) determining the amount of free glucose by reacting a mixture of sugar and glucose acceptor with dextransucrase; and (iii) comparing the amount of the free glucose of (i) with the amount of the free glucose of (ii).

### [Advantageous Effects]

A method of the present disclosure may be used to analyze activity of dextransucrase having transglycosylation activity for glucose acceptors by measuring a difference between the amount of free glucose after a sugar hydrolysis reaction by dextransucrase and the amount of free glucose after a sugar hydrolysis reaction and a transglycosylation reaction, thereby quickly and efficiently selecting or improving an enzyme having excellent dextransucrase activity having transglycosylation activity for glucose acceptors.

### [Description of Drawings]

FIG. 1 shows the amount of free glucose after a sugar hydrolysis reaction, the amount of free glucose after a sugar hydrolysis reaction and a transglycosylation reaction, and a difference therebetween, as measured with respect to a control enzyme;
FIG. 2 shows the amount of free glucose after a sugar hydrolysis reaction, the amount of free glucose after a sugar hydrolysis reaction and a transglycosylation reaction, and a difference therebetween, as measured with respect to an experimental enzyme; and
FIG. 3 shows transglycosylation patterns according to concentrations of control and experimental enzymes.

### [Best Mode]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

An aspect of the present disclosure provides a method of selecting dextransucrase having transglycosylation activity for glucose acceptors, the method including the steps of (i) determining the amount of free glucose by reacting sugar with dextransucrase; (ii) determining the amount of free glucose by reacting a mixture of sugar and a glucose acceptor with dextransucrase; and (iii) comparing the amount of the free glucose of (i) with the amount of the free glucose of (ii).

The existing determination of reducing sugar (DNS) is effective in confirming the degree of hydrolysis of sugar by dextransucrase, but there is a limitation in measuring transglycosylation activity. Specifically, there is also a limitation in directly confirming whether or not glucose acceptors have been glycosylated, and therefore, the present disclosure is characterized by developing a method of indirectly confirming glycosylation of glucose acceptors by comparing the amounts of free glucose.

As used herein, the term "dextransucrase", which is a kind of enzyme secreted from microorganisms, refers to an enzyme that produces polysaccharides, such as dextrans or oligosaccharides which are polymeric materials, by releasing glucose from sugar, and at the same time, by catalyzing a polymer polymerization reaction of glucose, or that exhibits transglycosylation activity capable of transferring glucose to stevia, polyphenol, *etc.* after releasing glucose from sugar, when glucose acceptors, such as stevia (steviol glycoside), polyphenol, *etc.,* exist in a substrate.

As used herein, the term "dextransucrase having transglycosylation activity for glucose acceptors" refers to dextransucrase that exhibits transglycosylation activity capable of transferring glucose to stevia, polyphenol, *etc.* after releasing glucose from sugar, when glucose acceptors, such as stevia, polyphenol, *etc.,* exist in a substrate, among the above-described dextransucrases. In other words, as used herein, the "dextransucrase having transglycosylation activity for glucose acceptors" may be dextransucrase having sugar hydrolysis activity and transglycosylation activity for glucose acceptors.

The "glucose acceptor" refers to a target substance to which glucose is transferred, and in the present disclosure, the glucose acceptor may be a natural product excluding glucose, specifically stevia or polyphenol, and more specifically stevia. However, the glucose acceptor is not limited as long as it is a natural product to which glucose is transferred.

As used herein, the term "stevia" refers to a sweet-tasting compound found in the leaves of stevia (*Stevia rebaudiana*), which is a plant belonging to the family Asteraceae, native to South America. Stevia is often used as a sweetener as a sugar substitute because it is not metabolized in the human body and does not induce a blood sugar response. The stevia is also called steviol glycoside.

With regard to the method of the present disclosure, step (i) may be determining the amount of free glucose by reacting sugar with dextransucrase.

The dextransucrase may be reacted by adding the dextransucrase at various concentrations, but is not limited thereto.

Specifically, in step (i), sugar as a substrate and a dextransucrase enzyme liquid are mixed and allowed to react, and then the amount of free glucose resulting from hydrolysis may be determined according to reaction times.

As the method of determining the amount of free glucose, any method known in the art may be used without limitation. For example, a method such as glucose oxidase (GOD)-peroxidase (POD) assay, determination of reducing sugar (DNS), liquid chromatography (LC) using a refractive index detector (RID), *etc.* may be used. In the present disclosure, the method of determining the amount of free glucose may be GOD-POD assay.

With regard to the present disclosure, step (ii) may be determining the amount of free glucose by reacting a mixture of sugar and glucose acceptor as a substrate with dextransucrase.

The dextransucrase may be reacted by adding the dextransucrase at the same concentrations as in step (i), but is not limited thereto.

Specifically, in step (ii), the mixture of sugar and a glucose acceptor and a dextransucrase enzyme liquid are mixed and allowed to react, and then the amount of free glucose resulting from hydrolysis may be determined according to reaction times.

The method of determining the amount of free glucose is the same as described above.

In the present disclosure, steps (i) and (ii) may be performed sequentially or simultaneously, but are not limited thereto.

With regard to the method of the present disclosure, step (iii) may be comparing the amount of free glucose of step (i) with the amount of free glucose of step (ii).

Specifically, the comparing may be comparing by subtracting the amount of free glucose of step (ii) from the amount of free glucose of step (i).

In the present disclosure, the selecting may be selecting dextransucrase having transglycosylation activity for glucose acceptors among dextransucrases, but is not limited thereto.

Specifically, with regard to the method of the present disclosure, in step (iii), the amount of free glucose of step (i) is compared with the amount of free glucose of step (ii), and as a result, when the amount of free glucose of step (ii) is reduced, as compared with the amount of free glucose of step (i), the dextransucrase is determined as dextransucrase having transglycosylation activity for glucose acceptors, and thus the dextransucrase may be selected as dextransucrase having transglycosylation activity for glucose acceptors.

Further, with regard to the method of the present disclosure, in step (iii), the amount of free glucose of step (i) is compared with the amount of free glucose of step (ii), and as a result, when the amount of free glucose of step (i) is similar to the amount of free glucose of step (ii), the dextransucrase may be determined as dextransucrase having no transglycosylation activity for glucose acceptors.

In the present disclosure, the selecting may be selecting dextransucrase having transglycosylation activity for glucose acceptors, but is not limited thereto. The selecting may also include measuring the activity thereof, but is not limited thereto.

Specifically, with regard to the method of the present disclosure, in step (iii), the amount of free glucose of step (i) is compared with the amount of free glucose of step (ii), and as a result, when the amount of free glucose of step (ii) is reduced, as compared with the amount of free glucose of step (i), the dextransucrase may be determined as dextransucrase having transglycosylation activity for glucose acceptors. Accordingly, it is possible to select dextransucrase having transglycosylation activity for glucose acceptors.

Further, with regard to the method of the present disclosure, in step (iii), the amount of free glucose of step (i) is compared with the amount of free glucose of step (ii), and as a result, when the amount of free glucose of step (ii) is reduced, as compared with the amount of free glucose of step (i), and the reduction level of the amount increases with increasing concentrations of dextransucrase or is higher than those of other dextransucrases, the transglycosylation activity of the dextransucrase may be measured through the reduction level of the amount.

In other words, when the reduction level in the amount of the free glucose of step (ii) is higher than that of the amount of free glucose of step (i), it is determined that the transglycosylation activity of dextransucrase is high, and thus it is possible to compare and/or measure the transglycosylation activities of dextransucrases which are different from each other.

The hydrolysis reaction of step (i) and the hydrolysis reaction and the transglycosylation reaction of step (ii) may be performed within 1 hour, but are not limited thereto. However, depending on the enzyme, when the hydrolysis reaction and the transglycosylation reaction continue even after 1 hour, the reactions may be performed without specific limitation in the time.

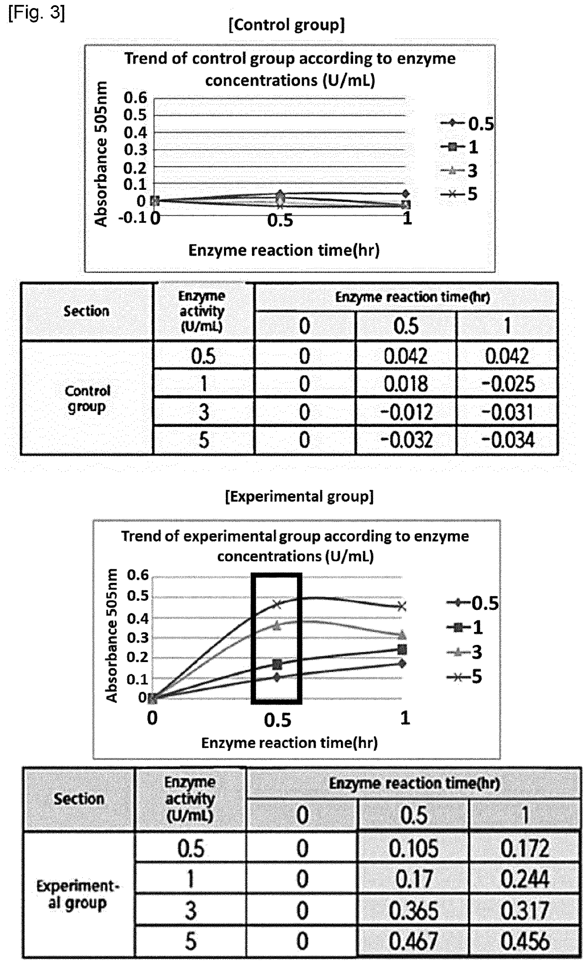

In the present disclosure, dextransucrase having high sugar hydrolysis activity and low transglycosylation activity while having high polysaccharide polymerization activity increases the amount of free glucose by releasing glucose after hydrolysis of sugar (a). However, when a glucose acceptor (stevia, polyphenol) is added (b), the glucose remains similar to (a) in a reactor, because the amount of glucose used in transglycosylation is very small, and thus the value of (a)-(b) approaches 0 (FIG. 3, left). However, dextransucrase having high sugar hydrolysis activity and high transglycosylation activity while having low polysaccharide polymerization activity increases the amount of free glucose by releasing glucose after hydrolysis of sugar (a), and when a glucose acceptor (stevia, polyphenol) is added (b), the free glucose is transferred to the glucose acceptor, and thus the value of (a)-(b) may be suggested as the amount of transglycosylated glucose. In this regard, it was observed that as the transglycosylation activity for the glucose acceptor (stevia, polyphenol) increased, the value of (a)-(b) also increased (FIG. 3, right).

Accordingly, an enzyme having the dextransucrase activity may be distinguished and selected by examining a difference in the amounts of free glucose after completing (a) sugar hydrolysis and (b) the polysaccharide polymerization or transglycosylation reaction of dextransucrase, as described above.

### [Mode for Invention]

Hereinafter, the present disclosure will be described in more detail with reference to Examples. However, these Examples are for illustrative purposes only, and it is apparent to those skilled in the art that the scope of the present disclosure is not intended to be limited by these Examples.

### Example 1: Evaluation of glycosyltransferase activity using difference between amount of free glucose after sugar hydrolysis and amount of free glucose after sugar hydrolysis and transglycosylation

Enzyme liquids with different dextransucrase activities (0.5 U/mL, 1.0 U/mL, 2.0 U/mL, 3.0 U/mL, based on determination of reducing sugar (DNS)) were each added to a 200 mM sugar solution and subjected to hydrolysis, and then the amount of free glucose was first determined, and the enzyme liquids with different dextransucrase activities were added to a sugar solution of the same concentration (200 mM) containing 200 mM stevia, respectively, and subjected to hydrolysis and transglycosylation, and the amount of free glucose was then determined. Assuming that a difference between the two determined amounts is the amount of pure transglycosylated glucose, it was examined whether the transglycosylation activity of the enzyme could be evaluated.

In detail, a sugar substrate alone and each control or experimental dextransucrase enzyme liquid (0.5 U/mL to 3.0 U/mL) were mixed and allowed to react, and then the amount of free glucose resulting from hydrolysis was first determined by way of a GOD (glucose oxidase)-POD (peroxidase) assay according to each reaction time (1). Further, a substrate mixture of sugar and stevia and each control or experimental dextransucrase enzyme liquid (0.5 U/mL to 3.0 U/mL) were mixed and allowed to react, and the amount of free glucose resulting from hydrolysis and transglycosylation was then determined by way of the GOD-POD assay according to each reaction time (2). Thereafter, the determined value of (2) was subtracted from the determined value of (1), and it was examined whether the amount of transglycosylated glucose had patterns according to the enzyme concentrations. At this time, *Leuconostoc mesenteroides* ATCC 13146-derived dextransucrase having sugar hydrolysis activity while having low transglycosylation activity and high polysaccharide polymerization activity was used as a negative control group, and *Lactobacillus mali* DSM20444-derived dextransucrase having sugar hydrolysis activity while having high transglycosylation activity and low polysaccharide polymerization activity was used as an experimental group.

### Example 2: Difference between amount of free glucose after sugar hydrolysis and amount of free glucose after sugar hydrolysis and transglycosylation by control enzyme

After a sugar hydrolysis reaction using *Leuconostoc mesenteroides* ATCC 13146-derived dextransucrase, the amount of free glucose was first determined by way of the GOD (glucose oxidase)-POD (peroxidase) assay (1). After hydrolysis/transglycosylation reactions using a substrate mixture of sugar and stevia, the amount of free glucose in the reaction mixture was then determined (2). A value of (1)-(2) was expected to be the amount of glucose used in the transglycosylation reaction, and the values of (1) and (2) were similar to each other, indicating that the value of (1)-(2) approached 0 (Table 1).

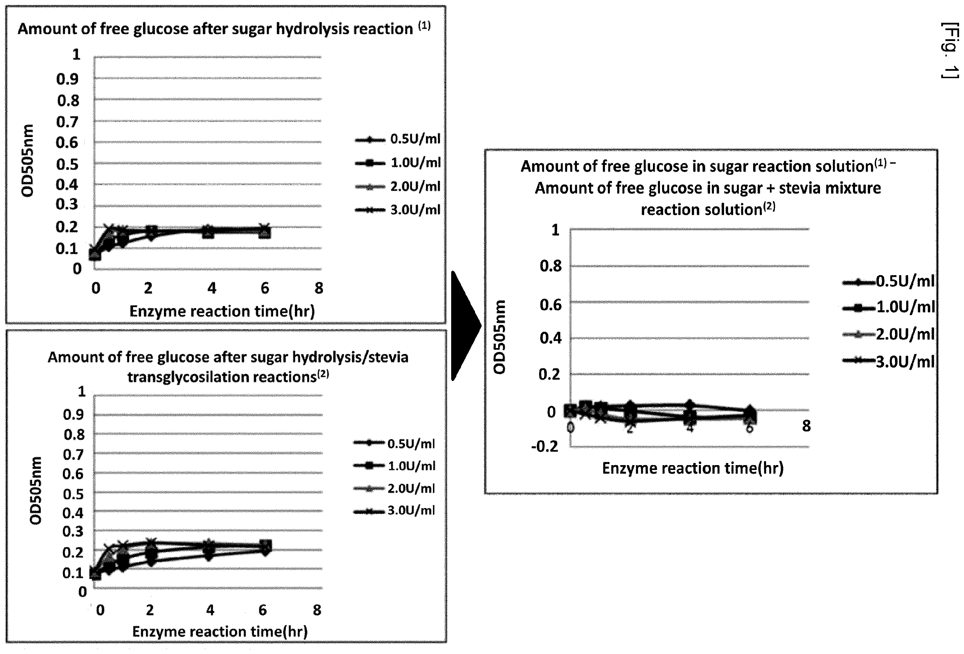

In detail, the control dextransucrase having sugar hydrolysis activity while having low transglycosylation activity and high polysaccharide polymerization activity showed no difference between the amount of free glucose after sugar hydrolysis and the amount of free glucose after sugar hydrolysis and transglycosylation (Table 1), indicating no patterns according to activities and reaction times (Table 2 and FIG. 1). The free glucose resulting from the sugar hydrolysis was immediately used as a substrate for the polysaccharide polymerization reaction, and thus the amount of free glucose in the reaction mixture was detected as being low.

**[Table 1]**

| Substrate | Enzyme activity (U/mL) | Enzyme reaction time (hr) | | | | | |
|---|---|---|---|---|---|---|---|
| | | 0 | 0.5 | 1 | 2 | 4 | 6 |
| Amount of free glucose after sugar hydrolysis reaction (1) | 0.5 | 0.000 | 0.042 | 0.058 | 0.091 | 0.123 | 0.119 |
| | 1 | 0.000 | 0.064 | 0.091 | 0.110 | 0.105 | 0.105 |
| | 2 | 0.000 | 0.091 | 0.102 | 0.103 | 0.102 | 0.099 |
| | 3 | 0.000 | 0.096 | 0.092 | 0.087 | 0.090 | 0.099 |
| Amount of free glucose after sugar hydrolysis/stevia transglycosylation reactions (2) | 0.5 | 0.000 | 0.023 | 0.036 | 0.065 | 0.095 | 0.120 |
| | 1 | 0.000 | 0.044 | 0.077 | 0.112 | 0.139 | 0.145 |
| | 2 | 0.000 | 0.081 | 0.121 | 0.149 | 0.149 | 0.142 |
| | 3 | 0.000 | 0.115 | 0.130 | 0.146 | 0.132 | 0.125 |

**[Table 2]**

| Enzyme activity (U/mL) | Reaction time (hr) | | | | | |
|---|---|---|---|---|---|---|
| | 0 | 0.5 | 1 | 2 | 4 | 6 |
| 0.5 | 0 | 0.019 | 0.022 | 0.026 | 0.028 | -0.001 |
| 1.0 | 0 | 0.02 | 0.014 | -0.002 | -0.034 | -0.04 |
| 2.0 | 0 | 0.01 | -0.019 | -0.046 | -0.047 | -0.043 |
| 3.0 | 0 | -0.019 | -0.038 | -0.059 | -0.042 | -0.026 |

### Example 3: Difference between amount of free glucose after sugar hydrolysis and amount of free glucose after sugar hydrolysis and transglycosylation by experimental enzyme

After a sugar hydrolysis reaction using *Lactobacillus mali* DSM20444-derived dextransucrase, the amount of free glucose was first determined by way of the GOD (glucose oxidase)-POD (peroxidase) assay (1). After hydrolysis/transglycosylation reactions using a substrate mixture of sugar and stevia, the amount of free glucose in the reaction mixture was then determined (2). A value of (1)-(2) was expected to be the amount of glucose used in the transglycosylation reaction, and it was observed that the value of (1)-(2) increased, as the transglycosylation activity was high (Table 3).

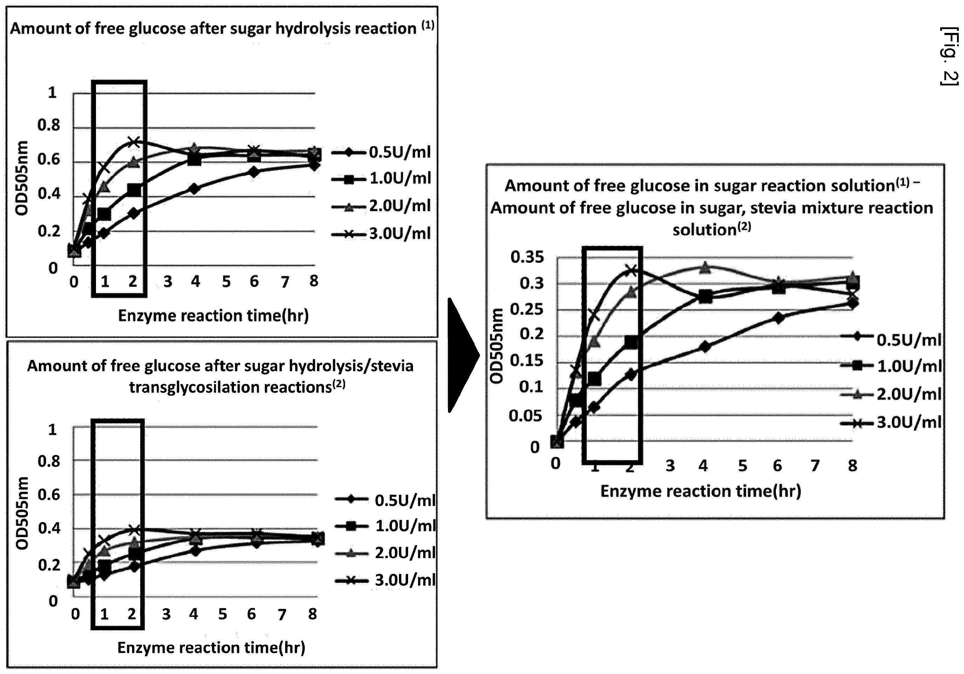

In detail, the dextransucrase having high sugar hydrolysis activity and high transglycosylation activity while having low polysaccharide polymerization activity showed a clear pattern according to each enzyme activity for the reaction time of 1 hour (Table 4 and FIG. 2).

**[Table 3]**

| Substrate | Enzyme activity (U/mL) | Enzyme reaction time (hr) | | | | | | |
|---|---|---|---|---|---|---|---|---|
| | | 0 | 0.5 | 1 | 2 | 4 | 6 | 8 |
| Amount of free glucose after sugar hydrolysis reaction (1) | 0.5 | 0 | 0.058 | 0.112 | 0.224 | 0.369 | 0.467 | 0.508 |
| | 1 | 0 | 0.129 | 0.217 | 0.358 | 0.536 | 0.557 | 0.56 |
| | 2 | 0 | 0.232 | 0.369 | 0.511 | 0.592 | 0.573 | 0.575 |
| | 3 | 0 | 0.289 | 0.472 | 0.62 | 0.548 | 0.57 | 0.533 |
| Amount of free glucose after sugar hydrolysis/stevia transglycosylation reactions (2) | 0.5 | 0 | 0.02 | 0.046 | 0.096 | 0.189 | 0.231 | 0.244 |
| | 1 | 0 | 0.05 | 0.097 | 0.168 | 0.258 | 0.263 | 0.256 |
| | 2 | 0 | 0.099 | 0.177 | 0.226 | 0.26 | 0.269 | 0.261 |
| | 3 | 0 | 0.153 | 0.23 | 0.295 | 0.273 | 0.273 | 0.252 |

**[Table 4]**

| Enzyme activity (U/mL) | Reaction time (hr) | | | | | | |
|---|---|---|---|---|---|---|---|
| | 0 | 0.5 | 1 | 2 | 4 | 6 | 8 |
| 0.5 | 0 | 0.038 | 0.066 | 0.128 | 0.18 | 0.236 | 0.264 |
| 1 | 0 | 0.079 | 0.12 | 0.19 | 0.278 | 0.294 | 0.304 |
| 2 | 0 | 0.133 | 0.192 | 0.285 | 0.332 | 0.304 | 0.314 |
| 3 | 0 | 0.136 | 0.242 | 0.325 | 0.275 | 0.297 | 0.281 |

### Example 4: Evaluation of reproducibility of glycosyltransferase activity using difference between amount of free glucose after sugar hydrolysis and amount of free glucose after sugar hydrolysis and transglycosylation

To evaluate the reproducibility of Example 1, experiments were performed by varying the stevia concentration and the enzyme concentration. Enzyme liquids with different dextransucrase activities (0.5 U/mL, 1.0 U/mL, 3.0 U/mL, 5.0 U/mL, based on determination of reducing sugar (DNS)) were each added to a 200 mM sugar solution and subjected to a hydrolysis reaction, and then the amount of free glucose was first determined, and the enzyme liquids with different dextransucrase activities were added to a sugar solution of the same concentration (200 mM) containing 100 mM stevia, respectively, and subjected to hydrolysis and transglycosylation reactions, and the amount of free glucose was then determined. Assuming that a difference between the two determined amounts is the amount of pure transglycosylated glucose, it was examined whether the transglycosylation activity of the enzyme could be evaluated.

In detail, a sugar substrate alone and each control or experimental dextransucrase enzyme liquid (0.5 U/mL to 5.0 U/mL) were mixed and allowed to react, and then the amount of free glucose resulting from hydrolysis was first determined by way of a GOD (glucose oxidase)-POD (peroxidase) assay according to each reaction time (1). Further, a substrate mixture of sugar and stevia and each control or experimental dextransucrase enzyme liquid (0.5 U/mL to 3 U/mL) were mixed and allowed to react, and the amount of free glucose resulting from hydrolysis and transglycosylation reactions was then determined by way of the GOD-POD assay according to each reaction time (2). Thereafter, the determined value of (2) was subtracted from the determined value of (1), and it was examined whether the amount of transglycosylated glucose had a pattern according to the enzyme concentrations (Tables 5 and 6).

**[Table 5]**

| Section | Enzyme activity (U/mL) | Enzyme reaction time (hr) | | |
|---|---|---|---|---|
| | | 0 | 0.5 | 1 |
| Control group | 0.5 | 0 | 0.042 | 0.042 |
| | 1 | 0 | 0.018 | -0.025 |
| | 3 | 0 | -0.012 | -0.031 |
| | 5 | 0 | -0.032 | -0.034 |

**[Table 6]**

| Section | Enzyme activity (U/mL) | Enzyme reaction time (hr) | | |
|---|---|---|---|---|
| | | 0 | 0.5 | 1 |
| Experimental group | 0.5 | 0 | 0.105 | 0.172 |
| | 1 | 0 | 0.17 | 0.244 |
| | 3 | 0 | 0.365 | 0.317 |
| | 5 | 0 | 0.467 | 0.456 |

As a result, the transglycosylation patterns were observed according to the enzyme concentration within 1 hour after the beginning of the reaction, which is the initial stage of the reaction, and it was confirmed that it is possible to select dextransucrase having transglycosylation activity for glucose acceptors (FIG. 3).

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and all changes and modifications that fall within metes and bounds of the claims or equivalents of such metes and bounds are therefore intended to be embraced by the claims.

## Claims

1. A method of selecting dextransucrase having transglycosylation activity for glucose acceptors, the method comprising steps of:
(i) determining the amount of free glucose by reacting sugar with dextransucrase;
(ii) determining the amount of free glucose by reacting a mixture of sugar and glucose acceptor with dextransucrase; and
(iii) comparing the amount of the free glucose of (i) with the amount of the free glucose of (ii).

2. The method of claim 1, wherein steps (i) and (ii) are performed sequentially or simultaneously.

3. The method of claim 1, wherein as a result of the comparison, when the amount of free glucose of (ii) is reduced, as compared with the amount of free glucose of (i), the dextransucrase is determined as dextransucrase having transglycosylation activity for glucose acceptors.

4. The method of claim 1, wherein the selecting is selecting dextransucrase having transglycosylation activity for glucose acceptors among dextransucrases.

5. The method of claim 1, wherein the selecting is selecting dextransucrase having transglycosylation activity for glucose acceptors or measuring the activity thereof.

6. The method of claim 1, wherein the glucose acceptor of step (ii) is stevia.
